(19) Europäisches Patentamt
European Patent Office
Office européen des brevets

(11) EP 4 692 316 A1

(12) EUROPEAN PATENT APPLICATION
published in accordance with Art. 153(4) EPC

(43) Date of publication:
**11.02.2026 Bulletin 2026/07**

(21) Application number: **25792063.7**

(22) Date of filing: **23.05.2025**

(51) International Patent Classification (IPC):
***C12N 5/00*** (2006.01) ***C08F 12/08*** (2006.01)
***C12M 1/12*** (2006.01) ***C12N 11/00*** (2006.01)
***C12N 11/08*** (2020.01)

(52) Cooperative Patent Classification (CPC):
**C12N 5/00**

(86) International application number:
**PCT/KR2025/095355**

(87) International publication number:
**WO 2025/244508 (27.11.2025 Gazette 2025/48)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH LA MA MD TN**

(30) Priority: **23.05.2024 KR 20240067363**
**22.05.2025 KR 20250066732**

(71) Applicant: **LG Chem, Ltd.**
**Yeongdeungpo-gu**
**Seoul 07336 (KR)**

(72) Inventors:
- **KIM, Minchae**
  **Daejeon 34122 (KR)**
- **KIM, Yeji**
  **Daejeon 34122 (KR)**
- **KIM, Jee Seon**
  **Daejeon 34122 (KR)**

(74) Representative: **Plasseraud IP**
**104 Rue de Richelieu**
**CS92104**
**75080 Paris Cedex 02 (FR)**

# (54) MICROCARRIER FOR CELL CULTURE, METHOD FOR PREPARING MICROCARRIER FOR CELL CULTURE, AND CELL CULTURE COMPOSITION USING SAME

(57) The present disclosure relates to a microcarrier for cell culture comprising polystyrene-based particles, a preparing method for the microcarrier for cell culture, and a cell culture composition using the same.



Processed by Luminess, 75001 PARIS (FR)

## Description

### [TECHNICAL FIELD]

This application claims the benefit of priority based on Korean Patent Application No. 10-2024-0067363 filed on May 23, 2024, and Korean Patent Application No. 10-2025-0066732 filed on May 22, 2025, the disclosures of which are incorporated herein by reference in their entirety.

The present disclosure relates to a microcarrier for cell culture, a preparing method for microcarrier for cell culture, and a cell culture composition using the same.

### [BACKGROUND]

As the biopharmaceutical and regenerative medical fields expand, there is a growing demand for large-scale cell culture technology capable of efficiently producing cells, tissues, microorganisms, and the like.

Adherent cells are cultured using micro carriers in a 3D bioreactor. The cells, culture medium and microcarrier are placed in the bioreactor, and the culture medium is agitated to bring the cells into contact with the micro carriers, so that the cells are adhered onto the surface of the micro carrier and cultured. Since the micro carrier used at this time provides a high surface area/volume ratio to which cells may attach and grow compared to 2D cultures, it is suitable for large-scale culture of cells.

Currently commercially available microcarriers have a density of about 1.05 to 1.3 g/cm$^3$, and cells have a density of about 1.05 to 1.2 g/cm$^3$. In this case, it is advantageous for attaching cells at the initial stage of culture in the bioreactor, but it is difficult to perform centrifugation when separating and recovering cells after culture, and a filtering method based on the microcarrier and cell size should be used. However, in this case, there are problems that the filter is clogged or the process requires a long time, and physical damage and contamination of cells may easily occur, and loss of cells may occur.

To address these issues, microcarriers have been fabricated from materials with densities lower than 1.0 g/cm$^3$ or higher than 1.3 g/cm$^3$. However, the attainable density range is limited, and it is difficult to achieve a sufficient yield of perfectly spherical microcarriers that are free from damage or breakage.

### [DETAILED DESCRIPTION OF THE INVENTION]

### [Technical Problem]

The present disclosure relates to a microcarrier for cell culture whose density is controlled so that it may be separated from cells by a density difference, while simultaneously exhibiting improved dispersibility in cell culture reactors or media and enhanced cell adhesion.

The present disclosure also relates to a preparing method for microcarrier for cell culture.

The present disclosure also relates to a cell culture composition using the microcarrier for cell culture.

### [Technical Solution]

According to an embodiment of the present disclosure, there is provided a microcarrier for cell culture comprising polystyrene-based particles that include a monomer compound represented by Chemical Formula 1 below, and having an apparent density of 0.99 g/cm$^3$ or more and 1.04 g/cm$^3$ or less:

[Chemical Formula 1]

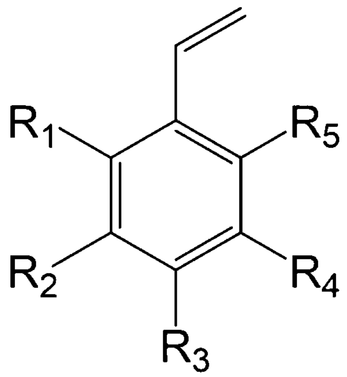

wherein in Chemical Formula 1, $R_1$ to $R_5$ are each independently hydrogen or an alkyl group having one or more carbon atoms, and at least one of $R_1$ to $R_5$ is an alkyl group having one or more carbon atoms.

According to another embodiment of the present disclosure, there is provided a preparing method for microcarrier for cell culture, comprising the step of: polymerizing and recovering polystyrene-based particles from a monomer mixture including a compound represented by the following Chemical Formula 1 below, and wherein the apparent density of the microcarrier for cell culture is in range of 0.99 g/cm$^3$ or more and 1.04 g/cm$^3$ or less:

[Chemical Formula 1]

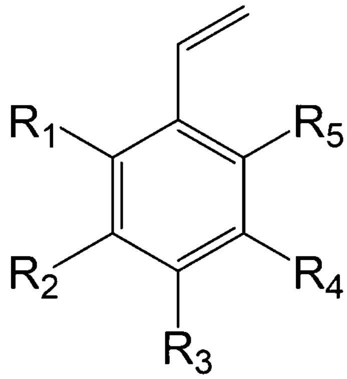

wherein in Chemical Formula 1, $R_1$ to $R_5$ are each independently hydrogen or an alkyl group having one or more carbon atoms, and at least one of $R_1$ to $R_5$ is an alkyl group having one or more carbon atoms.

According to yet another embodiment of the present disclosure, there is provided a cell-culture composition comprising cells and the above microcarrier for cell culture.

Now, a microcarrier for cell culture, a preparing method for microcarrier for cell culture, and a cell culture composition using the same according to specific embodiments of the present disclosure will be described in more detail.

Unless particularly mentioned herein, technical terms are used only to refer to particular embodiments and are not intended to limit the present disclosure.

Singular forms used herein include plural forms unless the context clearly indicates otherwise.

The term "including" or "comprising" as used herein specifies the presence of the stated features, regions, integers, steps, operations, elements, and/or components, but does not exclude the presence or addition of one or more other features, regions, integers, steps, operations, elements, components, and/or groups.

Further, the terms including ordinal numbers such as "a first", "a second", etc. are used only for the purpose of distinguishing one component from another component, and are not limited by the ordinal numbers. For instance, a first component may be referred to as a second component, or similarly, the second component may be referred to as the first component, without departing from the scope of the present disclosure.

The disclosure will be described in more detail.

According to an embodiment of the present disclosure, there may be provided a a microcarrier for cell culture comprising polystyrene-based particles that include a monomer compound represented by Chemical Formula 1, and having an apparent density of 0.99 g/cm$^3$ or more and 1.04 g/cm$^3$ or less.

The present inventors have found through experiments that in the case of the microcarrier for cell culture of this embodiment, including the compound represented by the Chemical Formula 1 as a monomer of the polystyrene-based polymer makes it possible to control the density of the final microcarrier for cell culture,, allowing separation based on the

density difference from cells, while simultaneously improving dispersibility in cell culture reactors or media and enhancing cell adhesion, and completed the present disclosure.

Conventional microcarriers for cell culture controlled the density of particles by encapsulating non-reactive low-density oil inside the particles, but there was a technical problem in that the low-density oil encapsulated inside leaked out when the particles were physically damaged.

Accordingly, the inventors confirmed that, by including the compound represented by the Chemical Formula 1 which has a density of 0.92 $g/cm^3$ or less as a monomer in the polystyrene-based polymer used for the microcarrier for cell culture, particle density may be regulated, enabling separation based on the density difference from cells, and simultaneously improving dispersibility in a bioreactor or culture medium.

Specifically, in one embodiment of the microcarrier for cell culture, the microcarrier may comprise polystyrene-based particles including a monomer compound represented by Chemical Formula 1 below:

[Chemical Formula 1]

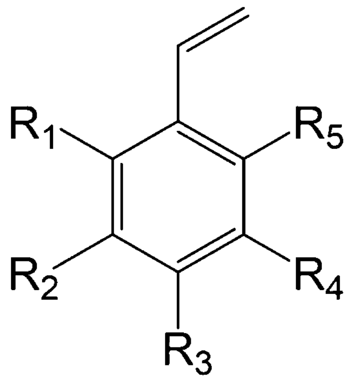

wherein in Chemical Formula 1, $R_1$ to $R_5$ are each independently hydrogen or an alkyl group having one or more carbon atoms, and at least one of $R_1$ to $R_5$ is an alkyl group having one or more carbon atoms.

By the microcarrier comprises the polystyrene-based particles including a monomer compound represented by Chemical Formula 1, the density of the final microcarrier for cell culture may be precisely controlled by adjusting the content of the compound represented by Chemical Formula 1, enabling separation based on the density difference from cells while simultaneously improving dispersibility in a cell culture reactor or medium.

Specifically, the compound represented by Chemical Formula 1 may include at least one compound selected from the group consisting of the compounds represented by Chemical Formulae 1-1 to 1-3 below:

[Chemical Formula 1-1]

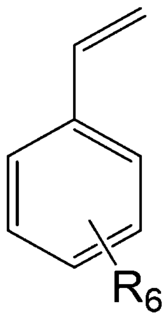

[Chemical Formula 1-2]

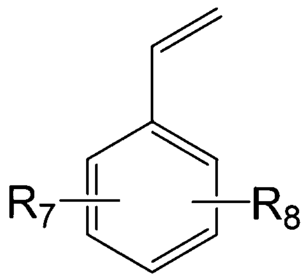

[Chemical Formula 1-3]

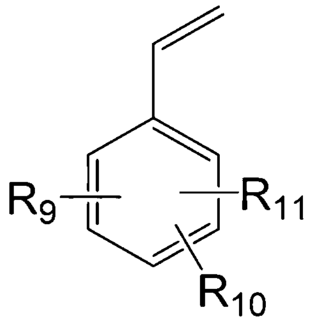

wherein in Chemical Formulas 1-1 to 1-3, $R_6$ to $R_{11}$ are each independently an alkyl group having one or more carbon atoms.

By including at least one compound selected from the group consisting of the compounds represented by Chemical Formulas 1-1 to 1-3 as a monomer of the polystyrene-based particles, the density of the microcarrier for cell culture may be precisely controlled by adjusting the content of the compound represented by Chemical formula 1, thereby enabling separation based on the density difference from cells and simultaneously improving dispersibility in a cell-culture reactor or medium.

More specifically, the compound represented by Chemical formula 1 may include at least one compound selected from the group consisting of the compound represented by the following Chemical Formulas 1-4 to 1-6:

[Chemical Formula 1-4]

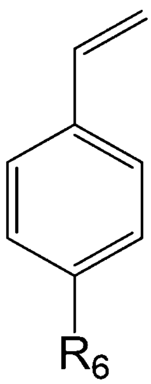

[Chemical Formula 1-5]

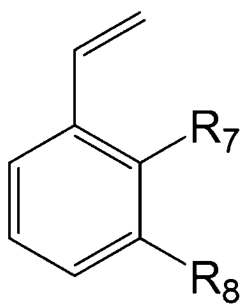

[Chemical Formula 1-6]

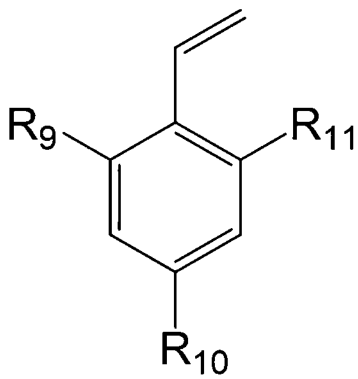

wherein in Chemical Formulas 1-4 to 1-6, $R_6$ to $R_{11}$ are each independently an alkyl group having one or more carbon atoms.

Meanwhile, the compound represented by Chemical Formula 1 may have a density of 0.92 g/cm$^3$ or less.

Specifically, the compound represented by Chemical Formula 1 may have a density of 0.92 g/cm$^3$ or less, 0.91 g/cm$^3$ or less, 0.906 g/cm$^3$ or less, 0.9 g/cm$^3$ or less, 0.89 g/cm$^3$ or less, 0.5 g/cm$^3$ or more, 0.6 g/cm$^3$ or more, 0.7 g/cm$^3$ or more, 0.8 g/cm$^3$ or more, 0.5 g/cm$^3$ or more and 0.92 g/cm$^3$ or less, 0.6 g/cm$^3$ or more and 0.92 g/cm$^3$ or less, 0.7 g/cm$^3$ or more and 0.92 g/cm$^3$ or less, 0.8 g/cm$^3$ or more and 0.92 g/cm$^3$ or less, 0.5 g/cm$^3$ or more and 0.91 g/cm$^3$ or less, 0.6 g/cm$^3$ or more and 0.91 g/cm$^3$ or less, 0.7 g/cm$^3$ or more and 0.91 g/cm$^3$ or less, 0.8 g/cm$^3$ or more and 0.91 g/cm$^3$ or less, 0.5 g/cm$^3$ or more and 0.906 g/cm$^3$ or less, 0.6 g/cm$^3$ or more and 0.906 g/cm$^3$ or less, 0.7 g/cm$^3$ or more and 0.906 g/cm$^3$ or less, 0.8 g/cm$^3$ or more and 0.906 g/cm$^3$ or less, 0.5 g/cm$^3$ or more and 0.9 g/cm$^3$ or less, 0.6 g/cm$^3$ or more and 0.9 g/cm$^3$ or less, 0.7 g/cm$^3$ or more and 0.9 g/cm$^3$ or less, 0.8 g/cm$^3$ or more and 0.9 g/cm$^3$ or less, 0.5 g/cm$^3$ or more and 0.89 g/cm$^3$ or less, 0.6 g/cm$^3$ or more and 0.89 g/cm$^3$ or less, 0.7 g/cm$^3$ or more and 0.89 g/cm$^3$ or less, 0.8 g/cm$^3$ or more and 0.89 g/cm$^3$ or less.

By the compound represented by Chemical Formula 1 having a density of 0.92 g/cm$^3$ or less, the density of the microcarrier for cell culture may be precisely controlled by adjusting the content of the compound represented by Chemical Formula 1, enabling separation based on the density difference from cells, while simultaneously improving dispersibility in a cell-culture bioreactor or medium.

For example, the compound represented by Chemical Formula 1 may include at least one compound selected from the group consisting of 4-methylstyrene, trimethylstyrene, 4-ethenyl-2-methyl-1-(2-methylpropyl)benzene, 1-ethenyl-2-methyl-4-(1-methylethyl)benzene, 1-ethenyl-2,3-dimethylbenzene, 4-(1,1-dimethylethyl)-2-ethenyl-1-methylbenzene, 2-ethenyl-4-methyl-1-(1-methylethyl)benzene, 1-ethenyl-2-(1-methylethyl)benzene, 2-tert-butylstyrene, 4-tert-butylstyrene, and 1-ethenyl-3-(1-methylethyl)benzene.

Meanwhile, the microcarrier for cell culture may comprise polystyrene-based particles. The polystyrene-based particles may include homopolymers of styrene-based monomers alone, or copolymers of styrene-based monomers with other monomers and crosslinking agents, and the styrene-based monomer may include a styrene monomer or derivatives thereof.

Preferably, the microcarrier for cell culture may be composed of polystyrene-based particles.

Specifically, the polystyrene-based particles may have the apparent density in a range of 0.99 g/cm$^3$ to 1.04 g/cm$^3$. By maintaining this low-density range, the cells and the microcarrier may be readily separated after culture through

the difference in sedimentation velocity under gravity when the microcarrier and cells are recovered.

When the density of the polystyrene-based particles exceeds 1.04 g/cm$^3$, the density difference between the cells and the microcarrier becomes small, which may make centrifugation difficult when separating and recovering the cells after culture. Conversely, when the density is less than 0.99 g/cm$^3$, the microcarrier may float only on the surface of the culture medium at the initial stage, making it difficult for cells to adhere.

The cells are adherent animal cells, without particular limitation thereto, and may include, for example, at least one cell selected from the group consisting of the fibroblasts, epithelial cells, osteoblasts, chondrocytes, hepatocytes, human umbilical cord blood-derived cells, human bone marrow-derived mesenchymal stem cells, CHO (Chinese hamster ovary) cells, kidney cells (HEK293, BHK21, MDCK, Vero cells, etc.), or a mixture of two or more thereof.

The density of the cell may be in range of 1.02 g/cm$^3$ or more and 1.1 g/cm$^3$ or less.

In addition, the density difference between the microcarrier for cell culture and the cells may be 0.02 g/cm$^3$ or more and 0.20 g/cm$^3$ or less. Satisfying a density difference of 0.02 g/cm$^3$ or more and 0.20 g/cm$^3$ or less allows the cells and the microcarrier to be easily separated after culture by exploiting the difference in sedimentation velocity under gravity when they are recovered, while also preventing the problem in which, at the early stage of culture, the microcarrier floats only on the surface of the culture medium, making cell attachment difficult.

The polystyrene-based particles may include the reaction product of a styrene-based monomer mixture and an ethylene-based unsaturated crosslinking agent.

Specifically, based on 100 parts by weight of the polystyrene-based particles, the reaction product of the styrene-based monomer mixture and the ethylene-based unsaturated crosslinking agent may be included in an amount of 90 parts by weight or more.

More specifically, based on 100 parts by weight of the polystyrene-based particles, the reaction product of the styrene-based monomer mixture and the ethylene-based unsaturated crosslinking agent may be included in an amount of 90 parts by weight or more and 100 parts by weight or less, 91 parts by weight or more and 100 parts by weight or less, or 95 parts by weight or more and 100 parts by weight or less.

Additionally, the polystyrene-based particles may include the compound represented by Chemical Formula 1 in amount of 51 parts by weight or more based on 100 parts by weight of the styrene-based monomer mixture. Specifically, the polystyrene-based particles may include the compound represented by Chemical Formula 1 in amount of 51 parts by weight or more and 100 parts by weight or less, 55 parts by weight or more and 100 parts by weight or less, 60 parts by weight or more and 100 parts by weight or less based on 100 parts by weight of the styrene-based monomer mixture.

By including the compound represented by Chemical Formula 1 in the above-mentioned amount based on 100 parts by weight of the styrene-based monomer mixture, the density of the final microcarrier for cell culture may be precisely controlled, enabling separation due to the density difference from the cells while simultaneously improving dispersibility in a cell-culture reactor or medium.

When the compound represented by Chemical Formula 1 is included in an excessively small amount based on 100 parts by weight of the styrene-based monomer mixture, the overall density of the polystyrene-based particles may become higher than the desired level.

In addition, the polystyrene-based particles may comprise the reaction product of the styrene-based monomer mixture and an ethylene-based unsaturated crosslinking agent, and the ethylene-based unsaturated crosslinking agent may be included in an amount of 60 parts by weight or more and 200 parts by weight or less based on 100 parts by weight of the styrene-based monomer mixture.

Specifically, the ethylene-based unsaturated crosslinking agent may be included in an amount of 60 parts by weight or more and 200 parts by weight or less, or 60 parts by weight or more and 150 parts by weight or less based on 100 parts by weight of the styrene-based monomer mixture.

When the ethylene-based unsaturated crosslinking agent is included in an excessively small amount based on 100 parts by weight of the styrene-based monomer mixture, the crosslinking density of the polystyrene-based polymer decreases, making it difficult to stably maintain the spherical shape of the particles.

Conversely, when the ethylene-based unsaturated crosslinking agent is included in an excessively large amount based on 100 parts by weight of the styrene-based monomer mixture, it becomes difficult to stably maintain the spherical shape of the particles, and the overall density of the polystyrene-based particles may exceed the targeted level.

Additionally, the polystyrene-based particles may include the compound represented by Chemical Formula 1 in an amount of 51 parts by weight or more and 200 parts by weight or less, based on 100 parts by weight of the ethylene-based unsaturated crosslinking agent.

Specifically, based on 100 parts by weight of the ethylene-based unsaturated crosslinking agent, the polystyrene-based particles may include the compound represented by Chemical Formula 1 in an amount of 51 parts by weight or more, 60 parts by weight or more, 65 parts by weight or more, 200 parts by weight or less, 180 parts by weight or less, 150 parts by weight or less, 51 parts by weight or more and 200 parts by weight or less, 60 parts by weight or more and 200 parts by weight or less, 65 parts by weight or more and 200 parts by weight or less, 51 parts by weight or more and 180 parts by weight or less, 60 parts by weight or more and 180 parts by weight or less, 65 parts by weight or more and 180 parts by

weight or less, 51 parts by weight or more and 150 parts by weight or less, 60 parts by weight or more and 150 parts by weight or less, 65 parts by weight or more and 150 parts by weight or less.

When the compound represented by Chemical Formula 1 is included in an excessively small amount based on 100 parts by weight of the ethylene-based unsaturated crosslinking agent, it is difficult for the particles to stably maintain a spherical shape, and there is a limitation in that the overall density of the polystyrene-based particles maynot readily be lowered to the targeted level.

When the compound represented by Chemical Formula 1 is included in an excessively large amount based on 100 parts by weight of the ethylene-based unsaturated crosslinking agent, the crosslinking density of the polystyrene-based polymer decreases, making it difficult to stably preserve the spherical morphology of the particles, and the overall density of the polystyrene-based particles falls below the target level.

Examples of the ethylene-based unsaturated crosslinking agent may include divinylbenzene.

Conventionally, a blowing agent has been added to lower the density of polystyrene particles and produce foamed styrene; however, this results in an excessively broad distribution of particle diameters and density ranges, making it difficult to secure a yield that falls within the range applicable for use as microcarriers.

The average diameter of the polystyrene-based particles may be 50 μm to 400 μm, or 60 μm to 390 μm, or 80 μm to 350 μm, or 90 μm to 300 μm. When the average diameter of the polystyrene-based particles falls within the aforementioned range, cell adhesion and culture performance are excellent. When the average diameter is less than 50 μm, the surface area available for cell culture becomes small, which may reduce culture efficiency; when it exceeds 400 μm, interactions among adherent cells decrease and the cell density in the bioreactor is lowered, which may likewise reduce culture efficiency.

The diameter of a polystyrene-based particle refers to the distance between two points where a straight line passing through the particle's center of gravity meets its outermost surface, and the average diameter of the polystyrene-based particles may be determined by measuring, under an optical microscope, the diameters of all the polystyrene-based particles included in the microcarrier for cell culture.

The polystyrene-based particles may be a group of individual particles having an average diameter of 50 μm to 400 μm, or 60 μm to 390 μm, and the individual fine particles included in the group may each have a diameter of, on average, 50 μm to 400 μm, or 60 μm to 390 μm. More specifically, 95 % or more, or 99 % or more of the individual fine particles contained in the group may have a diameter of 50 μm to 400 μm, or 60 μm to 390 μm.

In addition, the polystyrene-based particles may have a percentage of perfectly spherical particles without damage or breakage, as defined by the following equation, of more than 90% and 100% or less, 92% or more and 100% or less, 95% or more and 100% or less, 96% or more and 99% or less.

Percentage of perfectly spherical particles without damage or breakage (%) = (Number of polystyrene-based particles that are perfectly spherical without damage or breakage/Total number of polystyrene-based particles) $\times$ 100. [Equation]

The percentage of perfectly spherical particles without damage or breakage determined by the above equation may be obtained for the polystyrene-based particles by counting, via SEM, the number of particles that are perfectly spherical and free of damage or breakage among all particles and then calculating the percentage of those particles relative to the total.

Accordingly, the microcarrier for cell culture may include multiple polystyrene-based particles, and whether each of those particles is perfectly spherical without damage or breakage may be visually confirmed by SEM.

When the proportion of perfectly spherical particles without damage or fracture as defined by the above equation falls to 90% or less, the number of irregular particles with uneven, concave surfaces increases, and these irregular particles remain suspended in the cell culture medium, delivering physical impacts to the cells under cultivation, which may reduce cell culture efficiency to such an extent that successful cell culture becomes impossible.

Specifically, the D50 particle diameter of the microcarrier for cell culture may be in range of 100 μm to 300 μm, 100 μm to 250 μm, 120 μm to 250 μm, or 130 μm to 250 μm. When the average diameter of the microcarrier for cell culture falls within the foregoing range, cell attachment and culture performance are excellent. Conversely, when the D50 particle diameter of the microcarrier for cell culture is less than 100 μm, the available surface area for cell culture becomes limited, potentially reducing culture efficiency; when it exceeds 300 μm, interactions among adherent cells deteriorate and the cell density in the bioreactor decreases, which may likewise lead to diminished culture efficiency.

Meanwhile, the microcarrier for cell culture may include a cell attachment-inducing layer formed on the polystyrene-based particles.

The cell attachment-inducing layer includes cell-adhesive materials that provide sites where the cell's transmembrane proteins may bind, thereby enabling adherent cells to attach, spread, and be cultured stably.

The polymer forming the cell attachment-inducing layer is not particularly limited and may include one or more

polymer selected from the group consisting of gelatin, collagen, fibronectin, chitosan, poly L-lysine, vitronectin, peptides containing RGD, lignin, cationic dextran, dihydroxyphenylalanine , dopamine, norepinephrine, epinephrine, epigallocatechin, and derivatives thereof.

Additionally, the microcarrier for cell culture may optionally include a primer polymer layer formed between the polystyrene-based particle and the cell attachment-inducing layer. In one embodiment, the microcarrier for cell culture may comprise: the polystyrene-based particle; a primer polymer layer formed on the surface of the polystyrene-based particle; and a cell attachment-inducing layer formed on the surface of the primer polymer layer.

The primer polymer layer serves as an adhesive layer that enables the introduction of a functional polymer onto the non-functionalized polystyrene surface, thereby effectively introducing the polymer layer for cell attachment onto the microcarrier surface and maintaining its stability during culture.

Although not limited thereto, the primer polymer layer may include at least one polymer selected from the group consisting of catechol derivatives capable of inducing adhesion in an aqueous environment, such as L-dihydroxyphenylalanine (L-DOPA), dopamine, norepinephrine, epinephrine, epigallocatechin, and derivatives thereof.

Meanwhile, according to another embodiment of the present disclosure, there is provided a preparing method for microcarrier for cell culture, comprising the step of: polymerizing and recovering polystyrene-based particles from a monomer mixture including a compound represented by the following Chemical Formula 1 below, and wherein the apparent density of the microcarrier for cell culture is in range of 0.99 g/cm$^3$ or more and 1.04 g/cm$^3$ or less:

[Chemical Formula 1]

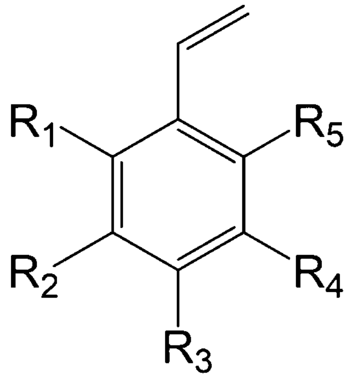

wherein in Chemical Formula 1, $R_1$ to $R_5$ are each independently hydrogen or an alkyl group having one or more carbon atoms, and at least one of $R_1$ to $R_5$ is an alkyl group having one or more carbon atoms.

In the preparing method for microcarrier for cell culture according to this embodiment, the descriptions of the polystyrene-based particles and the compound represented by Chemical Formula 1 encompass all of the aforementioned matters.

In the preparing method for microcarrier for cell culture according to this embodiment, the step of polymerizing and recovering polystyrene-based particles from a monomer mixture including a compound represented by the Chemical Formula 1 may include the step of performing a suspension polymerization reaction of the monomer composition comprising a styrene monomer and recovering the product of the suspension polymerization reaction.

Specifically, the compound represented by Chemical Formula 1 may include at least one compound selected from the group consisting of the compounds represented by Chemical Formulae 1-1 to 1-3 below:

[Chemical Formula 1-1]

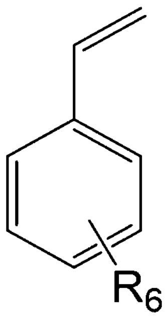

[Chemical Formula 1-2]

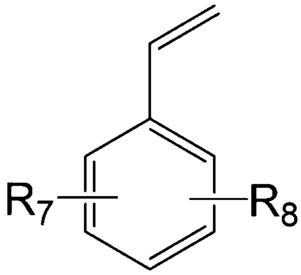

[Chemical Formula 1-3]

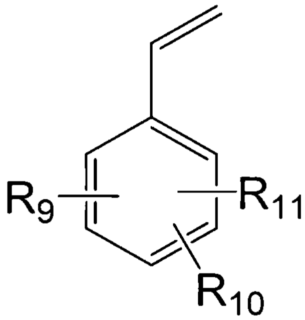

wherein in Chemical Formulas 1-1 to 1-3, $R_6$ to $R_{11}$ are each independently an alkyl group having one or more carbon atoms.

By including at least one compound selected from the group consisting of the compounds represented by Chemical Formulas 1-1 to 1-3 as a monomer of the polystyrene-based particles, the density of the microcarrier for cell culture may be precisely controlled by adjusting the content of the compound represented by Chemical formula 1, thereby enabling separation based on the density difference from cells and simultaneously improving dispersibility in a cell-culture reactor or medium.

More specifically, the compound represented by Chemical formula 1 may include at least one compound selected from the group consisting of the compound represented by the following Chemical Formulas 1-4 to 1-6:

[Chemical Formula 1-4]

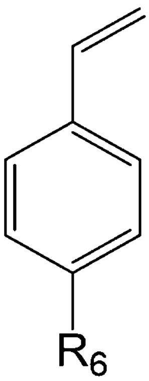

[Chemical Formula 1-5]

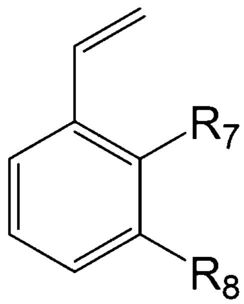

[Chemical Formula 1-6]

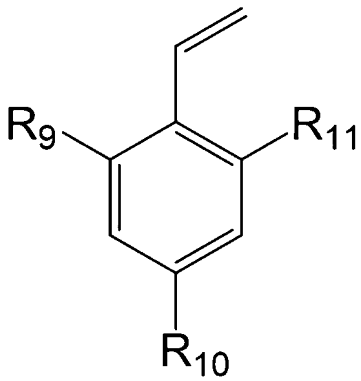

wherein in Chemical Formulas 1-4 to 1-6, $R_6$ to $R_{11}$ are each independently an alkyl group having one or more carbon atoms.

Meanwhile, the compound represented by Chemical Formula 1 may have a density of 0.92 g/cm$^3$ or less.

Specifically, the compound represented by Chemical Formula 1 may have a density of 0.92 g/cm$^3$ or less, 0.91 g/cm$^3$ or less, 0.906 g/cm$^3$ or less, 0.9 g/cm$^3$ or less, 0.89 g/cm$^3$ or less, 0.5 g/cm$^3$ or more, 0.6 g/cm$^3$ or more, 0.7 g/cm$^3$ or more, 0.8 g/cm$^3$ or more, 0.5 g/cm$^3$ or more and 0.92 g/cm$^3$ or less, 0.6 g/cm$^3$ or more and 0.92 g/cm$^3$ or less, 0.7 g/cm$^3$ or more and 0.92 g/cm$^3$ or less, 0.8 g/cm$^3$ or more and 0.92 g/cm$^3$ or less, 0.5 g/cm$^3$ or more and 0.91 g/cm$^3$ or less, 0.6 g/cm$^3$ or more and 0.91 g/cm$^3$ or less, 0.7 g/cm$^3$ or more and 0.91 g/cm$^3$ or less, 0.8 g/cm$^3$ or more and 0.91 g/cm$^3$ or less, 0.5 g/cm$^3$ or more and 0.906 g/cm$^3$ or less, 0.6 g/cm$^3$ or more and 0.906 g/cm$^3$ or less, 0.7 g/cm$^3$ or more and 0.906 g/cm$^3$ or less, 0.8 g/cm$^3$ or more and 0.906 g/cm$^3$ or less, 0.5 g/cm$^3$ or more and 0.9 g/cm$^3$ or less, 0.6 g/cm$^3$ or more and 0.9 g/cm$^3$ or less, 0.7 g/cm$^3$ or more and 0.9 g/cm$^3$ or less, 0.8 g/cm$^3$ or more and 0.9 g/cm$^3$ or less, 0.5 g/cm$^3$ or more and 0.89 g/cm$^3$ or less, 0.6 g/cm$^3$ or more and 0.89 g/cm$^3$ or less, 0.7 g/cm$^3$ or more and 0.89 g/cm$^3$ or less, 0.8 g/cm$^3$ or more and 0.89 g/cm$^3$ or less.

By the compound represented by Chemical Formula 1 having a density of 0.92 g/cm$^3$ or less, the density of the microcarrier for cell culture may be precisely controlled by adjusting the content of the compound represented by Chemical Formula 1, enabling separation based on the density difference from cells, while simultaneously improving dispersibility in a cell-culture bioreactor or medium.

For example, the compound represented by Chemical Formula 1 may include at least one compound selected from the group consisting of 4-methylstyrene, trimethylstyrene, 4-ethenyl-2-methyl-1-(2-methylpropyl)benzene, 1-ethenyl-2-methyl-4-(1-methylethyl)benzene, 1-ethenyl-2,3-dimethylbenzene, 4-(1,1-dimethylethyl)-2-ethenyl-1-methylbenzene, 2-ethenyl-4-methyl-1-(1-methylethyl)benzene, 1-ethenyl-2-(1-methylethyl)benzene, 2-tert-butylstyrene, 4-tert-butylstyrene, and 1-ethenyl-3-(1-methylethyl)benzene.

The polystyrene-based particles may include the reaction product of a styrene-based monomer mixture and an ethylene-based unsaturated crosslinking agent.

Specifically, based on 100 parts by weight of the polystyrene-based particles, the reaction product of the styrene-based monomer mixture and the ethylene-based unsaturated crosslinking agent may be included in an amount of 90 parts by weight or more.

More specifically, based on 100 parts by weight of the polystyrene-based particles, the reaction product of the styrene-based monomer mixture and the ethylene-based unsaturated crosslinking agent may be included in an amount of

90 parts by weight or more and 100 parts by weight or less, 91 parts by weight or more and 100 parts by weight or less, or 95 parts by weight or more and 100 parts by weight or less.

Additionally, the polystyrene-based particles may include the compound represented by Chemical Formula 1 in amount of 51 parts by weight or more based on 100 parts by weight of the styrene-based monomer mixture. Specifically, the polystyrene-based particles may include the compound represented by Chemical Formula 1 in amount of 51 parts by weight or more and 100 parts by weight or less, 55 parts by weight or more and 100 parts by weight or less, 60 parts by weight or more and 100 parts by weight or less based on 100 parts by weight of the styrene-based monomer mixture.

By including the compound represented by Chemical Formula 1 in the above-mentioned amount based on 100 parts by weight of the styrene-based monomer mixture, the density of the final microcarrier for cell culture may be precisely controlled, enabling separation due to the density difference from the cells while simultaneously improving dispersibility in a cell-culture reactor or medium.

When the compound represented by Chemical Formula 1 is included in an excessively small amount based on 100 parts by weight of the styrene-based monomer mixture, the overall density of the polystyrene-based particles may become higher than the desired level.

In addition, the polystyrene-based particles may comprise the reaction product of the styrene-based monomer mixture and an ethylene-based unsaturated crosslinking agent, and the ethylene-based unsaturated crosslinking agent may be included in an amount of 60 parts by weight or more and 200 parts by weight or less based on 100 parts by weight of the styrene-based monomer mixture.

Specifically, the ethylene-based unsaturated crosslinking agent may be included in an amount of 60 parts by weight or more and 200 parts by weight or less, or 60 parts by weight or more and 150 parts by weight or less based on 100 parts by weight of the styrene-based monomer mixture.

When the ethylene-based unsaturated crosslinking agent is included in an excessively small amount based on 100 parts by weight of the styrene-based monomer mixture, the crosslinking density of the polystyrene-based polymer decreases, making it difficult to stably maintain the spherical shape of the particles.

Conversely, when the ethylene-based unsaturated crosslinking agent is included in an excessively large amount based on 100 parts by weight of the styrene-based monomer mixture, it becomes difficult to stably maintain the spherical shape of the particles, and the overall density of the polystyrene-based particles may exceed the targeted level.

Additionally, the polystyrene-based particles may include the compound represented by Chemical Formula 1 in an amount of 51 parts by weight or more and 200 parts by weight or less, based on 100 parts by weight of the ethylene-based unsaturated crosslinking agent.

Specifically, based on 100 parts by weight of the ethylene-based unsaturated crosslinking agent, the polystyrene-based particles may include the compound represented by Chemical Formula 1 in an amount of 51 parts by weight or more, 60 parts by weight or more, 65 parts by weight or more, 200 parts by weight or less, 180 parts by weight or less, 150 parts by weight or less, 51 parts by weight or more and 200 parts by weight or less, 60 parts by weight or more and 200 parts by weight or less, 65 parts by weight or more and 200 parts by weight or less, 51 parts by weight or more and 180 parts by weight or less, 60 parts by weight or more and 180 parts by weight or less, 65 parts by weight or more and 180 parts by weight or less, 51 parts by weight or more and 150 parts by weight or less, 60 parts by weight or more and 150 parts by weight or less, 65 parts by weight or more and 150 parts by weight or less.

When the compound represented by Chemical Formula 1 is included in an excessively small amount based on 100 parts by weight of the ethylene-based unsaturated crosslinking agent, it is difficult for the particles to stably maintain a spherical shape, and there is a limitation in that the overall density of the polystyrene-based particles maynot readily be lowered to the targeted level.

When the compound represented by Chemical Formula 1 is included in an excessively large amount based on 100 parts by weight of the ethylene-based unsaturated crosslinking agent, the crosslinking density of the polystyrene-based polymer decreases, making it difficult to stably preserve the spherical morphology of the particles, and the overall density of the polystyrene-based particles falls below the target level.

Examples of the ethylene-based unsaturated crosslinking agent may include divinylbenzene.

More specifically, the performing a suspension polymerization reaction of the monomer composition comprising a styrene monomer may include: a step of mixing the monomer composition comprising a styrene monomer with an aqueous dispersion, applying shear force to homogenize the monomer composition into droplets in the aqueous dispersion; and a step of carrying out suspension polymerization of the homogenized monomer composition at a stirring speed of 300 rpm to 1000 rpm.

During the step of homogenizing the monomer composition into droplets in the aqueous dispersion, stirring may be performed at a speed of 300 rpm to 1000 rpm, or 400 rpm to 800 rpm.

In the step of carrying out a suspension polymerization of the homogenized monomer composition at a stirring speed of 300 rpm to 1000 rpm, or 400 rpm to 800 rpm, the particle structure of the polystyrene may further reduce the density of the microcarrier owing to the structure of the compound represented by Chemical Formula 1, while still enabling the production of microcarriers with a high proportion of intact, perfectly spherical particles without damage or breakage.

In the step of carrying out a suspension polymerization of the homogenized monomer composition at a stirring speed of 300 rpm to 1000 rpm, or 400 rpm to 800 rpm, the suspension polymerization conditions are not particularly limited; for example, the process may be conducted in a range of 50 °C to 100 °C for 3 hours to 18 hours.

Meanwhile, the preparing method for microcarrier for cell culture may further comprise the step of washing and drying steps after the step of polymerizing and recovering polystyrene-based particles from a monomer mixture containing the compound represented by Chemical Formula 1.

Specifically, the washing step may include filtering the reaction product through a 30 μm to 100 μm sieve, subsequently stirring it three to five times in distilled water at 50 °C to 75 °C, and then stirring it three to five times in ethanol at room temperature.

The drying step may include placing the washed product in a convection oven and drying it at room temperature or at a temperature of 80 °C or less. However, the present disclosure is not limited thereto, and any conventionally known drying method may be employed without particular restriction.

Meanwhile, the preparing method for microcarrier for cell culture may further include a step of coating a cell attachment-inducing layer on the polystyrene-based particles after the washing step and the drying step.

Optionally, the preparing method for microcarrier for cell culture may further include a step of coating a primer polymer layer on the surface of the polystyrene-based particles and coating a cell attachment-inducing layer on the polystyrene-based particles after the washing step and the drying step.

Specifically, the step of coating a primer polymer layer on the surface of the polystyrene-based particles may include immersing the polystyrene-based particles in a primer polymer solution for 1 to 10 hours, or 2 to 6 hours, or 3 to 5 hours.

The primer polymer layer is not particularly limited; however, it may include one or more polymer selected from the group consisting of catechol derivatives capable of inducing aqueous adhesion such as L-dihydroxyphenylalanine (L-DOPA), dopamine, norepinephrine, epinephrine, epigallocatechin, and derivatives thereof.

After recovering and drying the prepared polystyrene-based particles, coating the primer polymer layer renders the surface of the microcarrier hydrophilic, thereby enabling water dispersion, stably introducing a cell-adhesion-inducing layer, controlling the buoyancy of the microcarrier in the culture medium, and stably culturing cells in an adherent manner.

At this time, the ratio of the radius of the polystyrene-based particle to the thickness of the primer polymer layer may be 1:0.00001 to 1:0.01, or 1:0.0001 to 1:0.001.

Meanwhile, the preparing method for microcarrier for cell culture may further include, after coating the primer polymer layer on the surface of the polystyrene-based particle, a step of coating a cell attachment-inducing layer on the surface of the primer polymer layer.

The step of coating a cell attachment-inducing layer on the surface of the primer polymer layer may include coating with a solution including one or more compound selected from the group consisting of gelatin, collagen, fibronectin, chitosan, poly-L-lysine, vitronectin, peptides containing RGD, lignin, cationic dextran, and derivatives thereof.

A solution including one or more compound selected from the group consisting of gelatin, collagen, fibronectin, chitosan, poly-L-lysine, vitronectin, peptides containing RGD, lignin, cationic dextran, and derivatives thereof may act as an adhesion factor that bonds cells to the microcarriers; therefore, when the primer polymer layer is coated with such a solution, the attachment of cells to the microcarriers may be increased, making it more suitable for large-scale cell culture.

Specifically, the step of coating a cell attachment-inducing layer on the surface of the primer polymer layer may include immersing the product obtained from the step of coating a primer polymer layer on the surface of the polystyrene-based particles in a solution including one or more compound selected from the group consisting of gelatin, collagen, fibronectin, chitosan, poly-L-lysine, vitronectin, peptides including RGD, lignin, cationic dextran, and derivatives thereof, for 10 to 20 hours, or 15 to 20 hours, or 17 to 19 hours.

According to another embodiment of the disclosure a cell culture composition comprising cells and the microcarrier for cell culture of the aforementioned embodiment may be provided. All details described in the above embodiment regarding the microcarrier for cell culture are incorporated herein.

The cells are adherent animal cells, without particular limitation thereto, and may include, for example, at least one cell selected from the group consisting of the fibroblasts, epithelial cells, osteoblasts, chondrocytes, hepatocytes, human umbilical cord blood-derived cells, human bone marrow-derived mesenchymal stem cells, CHO (Chinese hamster ovary) cells, kidney cells (HEK293, BHK21, MDCK, Vero cells, etc.), or a mixture of two or more thereof.

The density of the cell may be in range of 1.02 g/$cm^3$or more and less than 1.1 g/$cm^3$.

In addition, the apparent density difference between the microcarrier for cell culture and the cells may be 0.02 g/$cm^3$ or more and 0.20 g/$cm^3$ or less. Satisfying a density difference of 0.02 g/$cm^3$ or more and 0.20 g/$cm^3$ or less allows the cells and the microcarrier to be easily separated after culture by exploiting the difference in sedimentation velocity under gravity when they are recovered, while also preventing the problem in which, at the early stage of culture, the microcarrier floats only on the surface of the culture medium, making cell attachment difficult.

The cell-culture composition may further include a medium solution. This medium solution may include various additives that sufficiently satisfy nutrients and environmental conditions such as pH, temperature, and osmotic pressure,

closely mimicking physiological conditions based on body fluids such as plasma or lymph. Any of a wide range of substances well known in the field of cell-culture technology may be used without restriction.

For example, in one embodiment, the microcarrier for cell culture has a lower density than the medium solution, so when it is introduced into the medium solution under agitation, it remains suspended within the solution. As the number of cells adhering to the surface of the low-density microcarrier increases, the density of the cell-laden microcarrier (hereinafter referred to as the "microcarrier-cell conjugate") gradually rises, causing it to settle within the medium solution.

Therefore, after treating the cell-laden microcarrier (microcarrier-cell conjugate) with a cell-detachment enzyme, it may be separated by centrifugation. By detaching the cells from the microcarrier-cell conjugate, the cultured cells may be readily collected.

**[Advantageous Effects]**

According to the present disclosure, a microcarrier for cell culture having high surface hydrophilicity and enhanced cell-attachment properties, improved dispersibility in a cell-culture reactor or medium, a preparing method for the microcarrier, and a cell-culture method using the same may be provided.

**[DETAILED DESCRIPTION OF THE EMBODIMENTS]**

Hereinafter, embodiments of the present disclosure will be described in more detail with reference to the following Examples. However, the following Examples are for illustrative purposes only, and are not intended to limit the scope of the present disclosure.

**Example 1**

Polyvinyl alcohol (molecular weight 85-124 K, 87-89 % hydrolysis) was dissolved in distilled water at 2 wt %, and 2 wt % of sodium chloride additive (based on the weight of distilled water) was added to the resulting aqueous dispersion, which was stirred at room temperature for 20 min.

A monomer composition was prepared by mixing styrene (Sigma-Aldrich), methylstyrene (Sigma-Aldrich, density 0.89-0.92 g/$cm^3$), and the cross-linker divinylbenzene (DVB) in a weight ratio of 2 : 3 : 5, thoroughly dissolving them, adding 2 wt % of the initiator V-65 to 60 g of the mixture (based on the total weight of monomers and cross-linker), and stirring for an additional 5 min.

A 1 L reactor was charged with 600 g of the aqueous dispersion, to which the monomer composition was added. Shear was applied to the aqueous dispersion and the monomer composition at 400 rpm at room temperature, dispersing the monomer composition into the aqueous phase as fine droplets and thereby homogenizing the mixture.

The homogenized mixture was stirred at 400 rpm and reacted under nitrogen purging at 85 °C for 6 h to produce polystyrene particles. The particles were washed three times with 60 °C distilled water and five times with ethanol, then recovered by drying in an 80 °C oven. The recovered polystyrene particles were used as microcarriers for cell culture.

Average diameter: 183 μm (D50 measured using PSA equipment)
Apparent density: 0.99-1.04 g/$cm^3$

**Example 2**

A microcarrier for cell culture was prepared in the same manner as in Example 1, except that the weight ratio of styrene, methylstyrene as monomers and divinylbenzene as cross-linker was adjusted to 1: 4: 5.

Average diameter: 196 μm (D50 measured using PSA equipment)
Apparent density: 0.99-1.02 g/$cm^3$

**Example 3**

A microcarrier for cell culture was prepared in the same manner as in Example 1, except that the weight ratio of styrene, methylstyrene as monomers and divinylbenzene as cross-linker was adjusted to 1: 5: 4, and the amount of initiator was increased to 3 wt%.

Average diameter: 183 μm (D50 measured using PSA equipment)
Apparent density: 0.99-1.02 g/$cm^3$

**Example 4**

A microcarrier for cell culture was prepared in the same manner as in Example 1, except that the weight ratio of ,methylstyrene as monomers and divinylbenzene as cross-linker was adjusted to 4:6, wherein the monomer does not include styrene.

Average diameter: 185 μm (D50 measured using PSA equipment)
Apparent density: 0.99-1.02 g/$cm^3$

**Example 5**

A microcarrier for cell culture was prepared in the same manner as in Example 1, except that the weight ratio of styrene, trimethylstyrene(Sigma-Aldrich, density: 0.906 g/$cm^3$) as monomers and divinylbenzene as cross-linker was adjusted to 2 : 3 : 5 .

Average diameter: 213 μm (D50 measured using PSA equipment)
Apparent density: 0.99-1.04 g/$cm^3$

**Example 6**

A microcarrier for cell culture was prepared in the same manner as in Example 5, except that the weight ratio of styrene, trimethylstyrene as monomers and divinylbenzene as cross-linker was adjusted to 1 : 4 : 5.

Average diameter: 180 μm (D50 measured using PSA equipment)
Apparent density: 0.99-1.04 g/$cm^3$

**Example 7**

A microcarrier for cell culture was prepared in the same manner as in Example 5, except that the weight ratio of trimethylstyrene as monomers and divinylbenzene as cross-linker was adjusted to 4:6.

Average diameter: 198 μm (D50 measured using PSA equipment)
Apparent density: 0.99-1.04 g/$cm^3$

**Example 8**

A microcarrier for cell culture was prepared in the same manner as in Example 7, except that the weight ratio of trimethylstyrene as monomers and divinylbenzene as cross-linker was adjusted to 5:5 .

Average diameter: 205 μm (D50 measured using PSA equipment)
Apparent density: 0.99-1.02 g/$cm^3$

**Example 9**

A microcarrier for cell culture was prepared in the same manner as in Example 8, except that the weight ratio of 4-tert-butyl styrene (Sigma-Aldrich, density: 0.875 g/$cm^3$), as monomers and divinylbenzene as cross-linker was adjusted to 5:5 .

Average diameter: 221 μm (D50 measured using PSA equipment)
Apparent density: 0.99-1.04 g/$cm^3$

**Example10**

A microcarrier for cell culture was prepared in the same manner as in Example 1, except that the weight ratio of styrene, 4-tert-butylstyrene as monomers and divinylbenzene as cross-linker was adjusted to 2 : 3 : 5.

Average diameter: 231 μm (D50 measured using PSA equipment)
Apparent density: 0.99-1.04 g/$cm^3$

**Examplell**

A microcarrier for cell culture was prepared in the same manner as in Example 10, except that the weight ratio of styrene, 4-tert-butylstyrene as monomers and divinylbenzene as cross-linker was adjusted to 1:5:4 , and the amount of initiator was increased to 3 wt%.

Average diameter: 183 μm (D50 measured using PSA equipment)
Apparent density: 0.99-1.04 g/$cm^3$

**Example12**

A microcarrier for cell culture was prepared in the same manner as in Example 2, except that the weight ratio of 2,4-Dimethylstyrene (Sigma-Aldrich, density: 0.906 g/$cm^3$), methylstyrene as monomers and divinylbenzene as cross-linker was adjusted to 1:4:5.

Average diameter: 205 μm (D50 measured using PSA equipment)
Apparent density: 0.99-1.04 g/$cm^3$

**Example 13**

A microcarrier for cell culture was prepared in the same manner as in Example 12, except that the weight ratio of 2,4-Dimethylstyrene (Sigma-Aldrich), methylstyrene as monomers and divinylbenzene as cross-linker was adjusted to 1:5:4, and the amount of initiator was increased to 3 wt%.

Average diameter: 198 μm (D50 measured using PSA equipment)
Apparent density: 0.99-1.02 g/$cm^3$

**Example 14**

A microcarrier for cell culture was prepared in the same manner as in Example 12, except that the weight ratio of 2,4-Dimethylstyrene, trimethylstyrene as monomers and divinylbenzene as cross-linker was adjusted to 1:4:5 .

Average diameter: 187 μm (D50 measured using PSA equipment)
Apparent density: 0.99-1.02 g/$cm^3$

**Example 15**

A microcarrier for cell culture was prepared in the same manner as in Example 12, except that the weight ratio of 2,4-Dimethylstyrene, 4-tert-butylstyrene as monomers and divinylbenzene as cross-linker was adjusted to 1:5:4, and the amount of initiator was increased to 3 wt%.

Average diameter: 218 μm (D50 measured using PSA equipment)
Apparent density: 0.99-1.04 g/$cm^3$

[Table 1]

| | styrene (g) | 4-methyl styrene (g) | 2,4,6-trimethyl styrene (g) | 4-tert-butyl styrene (g) | 2,4-dimethyl styrene (g) | DVB (g) | V-65 (g) |
|---|---|---|---|---|---|---|---|
| Example 1 | 12 | 18 | - | - | - | 30 | 1.2 |
| Example 2 | 6 | 24 | - | - | - | 30 | 1.2 |
| Example3 | 6 | 30 | - | - | - | 24 | 1.8 |
| Example4 | 0 | 24 | - | - | - | 36 | 1.2 |
| Example5 | 12 | - | 18 | - | - | 30 | 1.2 |

(continued)

| | styrene (g) | 4-methyl styrene (g) | 2,4,6-trimethyl styrene (g) | 4-tert-butyl styrene (g) | 2,4-dimethyl styrene (g) | DVB (g) | V-65 (g) |
|---|---|---|---|---|---|---|---|
| Example6 | 6 | - | 24 | - | - | 30 | 1.2 |
| Example7 | 0 | - | 24 | - | - | 36 | 1.2 |
| Example8 | 0 | - | 30 | - | - | 30 | 1.2 |
| Example9 | 0 | - | - | 30 | - | 30 | 1.2 |
| Example 10 | 12 | - | - | 18 | - | 30 | 1.2 |
| Example 11 | 6 | - | - | 30 | - | 24 | 1.8 |
| Example 12 | - | 24 | - | - | 6 | 30 | 1.2 |
| Example 13 | - | 30 | - | - | 6 | 24 | 1.8 |
| Example 14 | - | - | 24 | - | 6 | 30 | 1.2 |
| Example 15 | - | - | - | 30 | 6 | 24 | 1.8 |

**Comparative Example 1**

Polyvinyl alcohol (molecular weight 85-124 K, 87-89 % hydrolysis) was dissolved in distilled water at 2 wt %, and 2 wt % sodium chloride additive (based on the weight of distilled water) was introduced into the aqueous dispersion, followed by stirring at room temperature for 20 minutes.

Styrene as the monomer and divinylbenzene as the cross-linker were mixed at a weight ratio of 1:1. Isopar M (a mixture of iso-alkanes with carbon numbers 12-14 and 13-16; density 0.79 g/cm$^3$) was added as oil so that the oil content (based on the total weight of the monomer, cross-linker, and oil) reached 10 wt %. After complete dissolution, 120 g of this mixture were taken, 2 wt % of initiator V-65 (based on the total weight of the monomer and cross-linker) was added, and the mixture was stirred for an additional 5 minutes to obtain the monomer composition.

A 1 L reactor was charged with 600 g of the aqueous dispersion, to which the monomer composition was added. Shear was applied to the aqueous dispersion and the monomer composition at 400 rpm at room temperature, dispersing the monomer composition into the aqueous phase as fine droplets and thereby homogenizing the mixture.

The homogenized mixture was stirred at 400 rpm and reacted under nitrogen purging at 85 °C for 6 h to produce polystyrene particles. The particles were washed three times with 60 °C distilled water and five times with ethanol, then recovered by drying in an 80 °C oven. The recovered polystyrene particles were used as microcarriers for cell culture.

The physical properties of the polystyrene particles are as follows.

Average diameter: 194 μm (D50 measured using PSA equipment)
Apparent density: over 1.003 g/cm$^3$
Pore diameter: 0.05 μm - 4 μm

**Comparative Example 2**

A microcarrier for cell culture was prepared in the same manner as in Comparative Example 1, except that Isopar M (a mixture of iso-alkanes with carbon numbers 12-14 and 13-16; density 0.79 g/cm$^3$) was added as oil so that the oil content (based on the total amount of monomer, crosslinking agent, and oil) was 14 wt%.

The physical properties of the polystyrene particles are as follows.

Average diameter: 190.6 μm (D50 measured using PSA equipment)
Apparent density: 0. 98 ~ 0.99 g/cm$^3$
Pore diameter: 0.05 μm - 4 μm

**Comparative Example 3**

A microcarrier for cell culture was prepared in the same manner as in Comparative Example 1, except that Isopar M (a mixture of iso-alkanes with carbon numbers 12-14 and 13-16; density 0.79 g/cm$^3$) was added as oil so that the oil content (based on the total amount of monomer, crosslinking agent, and oil) was 20 wt%.

The physical properties of the polystyrene particles are as follows.

Average diameter: 188.07 μm (D50 measured using PSA equipment)
Apparent density: 0. 97 ~ 0.98 g/$cm^3$
Pore diameter: 0.05 μm - 4 μm

**Comparative Example 4**

A microcarrier for cell culture was prepared in the same manner as in Example 1, except that the weight ratio of styrene,alone as monomers and divinylbenzene as cross-linker was adjusted to 1: 1..

Average diameter: 198 μm (D50 measured using PSA equipment)
Apparent density: > 1.04 g/$cm^3$

**Reference Example** 1

A microcarrier for cell culture was prepared in the same manner as in Example 1, except that the weight ratio of each monomer as shown in Table 2.

Average diameter: 204.5μm (D50 measured using PSA equipment)
Apparent density: > 1.04 g/$cm^3$

[Table 2]

| | styrene (g) | 4-methyl styrene (g) | 2,4,6-trimethyl styrene (g) | 4-tert-butyl styrene (g) | 2,4-dimethyl styrene (g) | DVB (g) | IsoparM (g) | V-65 (g) |
|---|---|---|---|---|---|---|---|---|
| Comparative Example 1 | 27 | - | - | - | - | 27 | 6 | 1.2 |
| Comparative Example 2 | 25.8 | - | - | - | - | 25.8 | 8.4 | 1.2 |
| Comparative Example 3 | 24 | - | - | - | - | 24 | 12 | 1.2 |
| Comparative Example 4 | 30 | - | - | - | - | 30 | - | 1.2 |
| Reference Example 1 | 28.5 | 1.5 | - | - | - | 30 | - | 1.2 |

**Experimental Example: Measurement of Physical Properties of Microcarriers for Cell Culture**

For the microcarriers for cell culture obtained in the Examples and Comparative Example, their physical properties were measured by the following methods, and the results are shown in Table 3.

**Experiment 1. Average Particle Size (unit:** μm)

For the microcarriers for cell culture obtained in the Examples and Comparative Example, the particles were dispersed in ethanol at a 10 wt% level, after which the D50 particle diameter (the particle size corresponding to the cumulative distribution percentage reaching 50%) was measured using a PSA (Particle Size Analysis) instrument.

**Experiment 2. Apparent density (unit: g/$cm^3$)**

For the microcarrier for cell culture prepared in the Examples and Comparative Example, the apparent density was evaluated at room temperature (25 °C) and atmospheric pressure (1 atm) by adding the particles to ethanol aqueous solutions with densities of 0.985 g/$cm^3$, 0.99 g/$cm^3$, and 0.997 g/$cm^3$ and to glycerol aqueous solutions with densities of 1.02 g/$cm^3$ and 1.04 g/$cm^3$, and observing whether they floated or sank according to the criteria below.

1) 0.985 g/$cm^3$ < d < 0.99 g/$cm^3$ (greater than 0.985 g/$cm^3$ and less than 0.99 g/$cm^3$)
Sinks in an ethanol aqueous solution with a density of 0.985 g/$cm^3$ and floats in an ethanol aqueous solution with a

density of 0.99 g/$cm^3$.
2) 0.99 g/$cm^3$ < d < 1.02 g/$cm^3$ (greater than 0.99 g/$cm^3$ and less than 1.02 g/$cm^3$ )
Sinks in an ethanol aqueous solution with a density of 0.99 g/$cm^3$ and floats in a glycerol aqueous solution with a density of 1.02 g/$cm^3$.
3) 0.99 g/$cm^3$ < d < 1.04 g/$cm^3$ (greater than 0.99 g/$cm^3$ and less than 1.04 g/$cm^3$ )
Sinks in an ethanol aqueous solution with a density of 0.99 g/$cm^3$ and floats in a glycerol aqueous solution with a density of 1.04 g/$cm^3$.
4) 0.99 g/$cm^3$ < d < 1.003 g/$cm^3$ (greater than 0.99 g/$cm^3$ and less than 1.003 g/$cm^3$)
Sinks in distilled water with a density of 0.99 g/$cm^3$ and floats in a cell culture medium with a density of 1.003 g/$cm^3$.
5) d > 1.04 g/$cm^3$ (greater than 1.04 g/$cm^3$)
Sinks in a glycerol aqueous solution with a density of 1.04 g/$cm^3$.

**Experiment 3. Dispersibility of particles in the bioreactor**

Prior to cultivation, the required amount of the microcarrier for cell culture was pre-dispersed with medium in a 20 mL glass vial and wetted for approximately 10-18 hours. The pre-dispersed microcarrier was filtered through a cell strainer, transferred along with 60 mL of medium into a 100 mL 3D bioreactor, and agitated for 24 hours under actual cell culture conditions (37 °C, 5 % $CO_2$ incubator, bioreactor stirring at 25 rpm).
After 24 hours, particles that remained undispersed and were floating at the medium's upper interface were removed by filtration. The particles dispersed within the bioreactor were thoroughly washed with DPBS or water, dried, and weighed. The dispersion rate was calculated as the percentage of the dispersed particle weight relative to the total weight, and evaluation was conducted according to the criteria below.

Excellent : Dispersibility of 80% or greater and up to 100%
Good: Dispersibility of 60 % or more and less than 80 %
Poor: Dispersibility of less than 60 %

**Experiment 4. Possibility of separation by density difference**

For the microcarrier for cell culture prepared in the Examples and Comparative Example, the possibility of separating the cells from the microcarriers for cell culture by means of density difference was evaluated under ambient temperature (25 °C) and atmospheric pressure (1 atm) by centrifuging a mixture of the cell culture medium and the microcarriers as follows.

O: No particles sank at the bottom after centrifugation.
X: Particles sank at the bottom after centrifugation.

[Table 3]

| | Average Particle Size ($\mu$m, D50) | Apparent density (g/$cm^3$) | Dispersibility (%) | Possibility of separation by density difference |
|---|---|---|---|---|
| Example 1 | 183 | 0.99 - 1.04 | Good | O |
| Example 2 | 196 | 0.99 - 1.02 | Good | O |
| Example3 | 183 | 0.99 - 1.02 | Good | O |
| Example4 | 185 | 0.99 - 1.02 | Excellent | O |
| Example5 | 213 | 0.99 - 1.04 | Excellent | O |
| Example6 | 180 | 0.99 - 1.04 | Excellent | O |
| Example7 | 198 | 0.99 - 1.04 | Excellent | O |
| Example8 | 205 | 0.99 - 1.02 | Excellent | O |
| Example9 | 221 | 0.99 - 1.04 | Excellent | O |
| Example10 | 231 | 0.99 - 1.04 | Good | O |
| Example11 | 183 | 0.99 - 1.04 | Excellent | O |

(continued)

| | Average Particle Size ($\mu$m, D50) | Apparent density (g/cm$^3$) | Dispersibility (%) | Possibility of separation by density difference |
|---|---|---|---|---|
| Example 12 | 205 | 0.99 - 1.04 | Good | O |
| Example 13 | 198 | 0.99 - 1.02 | Excellent | O |
| Example 14 | 187 | 0.99 - 1.04 | Good | O |
| Example 15 | 218 | 0.99 - 1.04 | Excellent | O |
| Comparative Example 1 | 194 | > 1.003 | Good | X |
| Comparative Example 2 | 191 | 0.98 - 0.99 | Poor | O |
| Comparative | 191 | 0.97 - 0.98 | Poor | O |
| Example 3 | | | | |
| Comparative Example 4 | 198 | >1.04 | Poor | X |
| Reference Example 1 | 204.5 | >1.04 | Poor | X |

As shown in Table 3, the microcarrier for cell culture according to the embodiment has a low density of 1.04 g/cm$^3$ or less, making it suitable for cell culture; as a result, improved particle dispersibility under culture conditions was confirmed, and separation of the microcarrier by density difference was possible.
In contrast, the microcarrier for cell culture of Comparative Example 1 could not be separated by density difference.
Moreover, because the microcarriers of Comparative Examples 2 and 3 contain low-density oil within the particles, not only was particle dispersibility under culture conditions reduced, but there was also a possibility that the encapsulated low-density oil could leak out due to damage to the microcarriers during culture.
In the case of Comparative Example 4, particle dispersibility under culture conditions deteriorated, and separation of the microcarrier by density difference was also impossible.

**Claims**

1. A microcarrier for cell culture, comprising polystyrene-based particles that include a monomer compound represented by Chemical Formula 1, and

   having an apparent density of 0.99 g/cm$^3$ or more and 1.04 g/cm$^3$ or less:

   [Chemical Formula 1]

   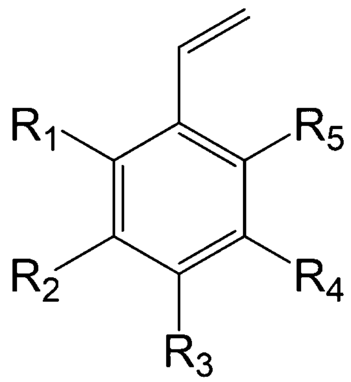

   wherein in Chemical Formula 1,
   $R_1$ to $R_5$ are each independently hydrogen or an alkyl group having one or more carbon atoms, and
   at least one of $R_1$ to $R_5$ is an alkyl group having one or more carbon atoms.

2. The microcarrier for cell culture of claim 1, wherein:

the compound represented by Chemical Formula 1 comprises at least one compound selected from the group consisting of the compounds represented by Chemical Formulae 1-1 to 1-3:

[Chemical Formula 1-1]

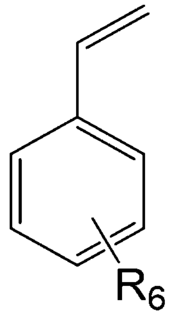

[Chemical Formula 1-2]

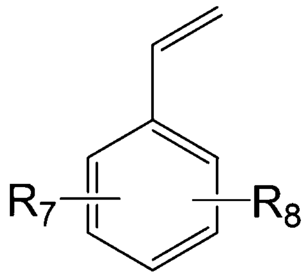

[Chemical Formula 1-3]

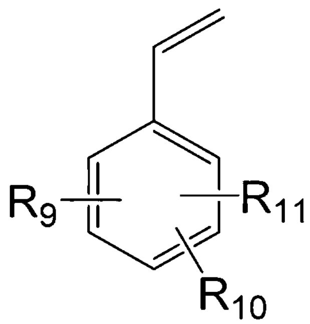

wherein in Chemical Formulas 1-1 to 1-3,
$R_6$ to $R_{11}$ are each independently an alkyl group having one or more carbon atoms.

**3.** The microcarrier for cell culture of claim 1, wherein:
the compound represented by Chemical Formula 1 have a density of 0.92 g/cm$^3$ or less.

**4.** The microcarrier for cell culture of claim 1, wherein:

the polystyrene-based particles comprise the reaction product of a styrene-based monomer mixture and an ethylene-based unsaturated crosslinking agent, and
the reaction product of the styrene-based monomer mixture and the ethylene-based unsaturated crosslinking agent is included in an amount of 90 parts by weight or more based on 100 parts by weight of the polystyrene-based particles.

**5.** The microcarrier for cell culture of claim 1, wherein: comprises a cell attachment-inducing layer formed on the polystyrene-based particles.

**6.** The microcarrier for cell culture of claim 1, wherein:

the polystyrene-based particles comprise the reaction product of a styrene-based monomer mixture and an ethylene-based unsaturated crosslinking agent, and
the compound represented by Chemical Formula 1 is included in an amount of 51 parts by weight or more based on 100 parts by weight of the styrene-based monomer mixture.

**7.** The microcarrier for cell culture of claim 1, wherein:

the polystyrene-based particles comprise the reaction product of a styrene-based monomer mixture and an ethylene-based unsaturated crosslinking agent, and
the compound represented by Chemical Formula 1 is included in an amount of 51 parts by weight or more and 200 parts by weight or less based on 100 parts by weight of the ethylene-based unsaturated crosslinking agent.

**8.** The microcarrier for cell culture of claim 1, wherein:
D50 particle diameter of the microcarrier for cell culture is in range of 100 μm to 300 μm.

**9.** A preparing method for microcarrier for cell culture, comprising the step of:

polymerizing and recovering polystyrene-based particles from a monomer mixture including a compound represented by the following Chemical Formula 1, and
wherein the apparent density of the microcarrier for cell culture is in range of 0.99 g/cm$^3$ or more and 1.04 g/cm$^3$ or less:

[Chemical Formula 1]

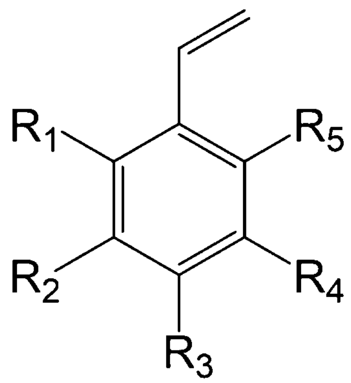

wherein in Chemical Formula 1,
$R_1$ to $R_5$ are each independently hydrogen or an alkyl group having one or more carbon atoms, and
at least one of $R_1$ to $R_5$ is an alkyl group having one or more carbon atoms.

**10.** The preparing method for microcarrier for cell culture of claim 9, wherein:

the compound represented by Chemical Formula 1 comprises at least one compound selected from the group consisting of the compounds represented by Chemical Formulae 1-1 to 1-3:

[Chemical Formula 1-1]

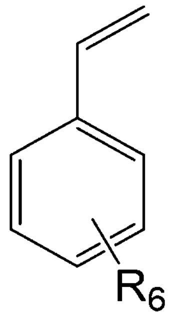

[Chemical Formula 1-2]

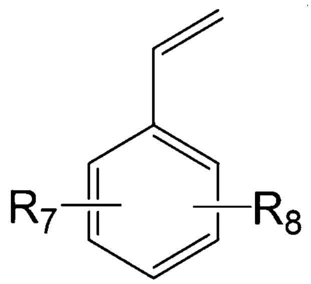

[Chemical Formula 1-3]

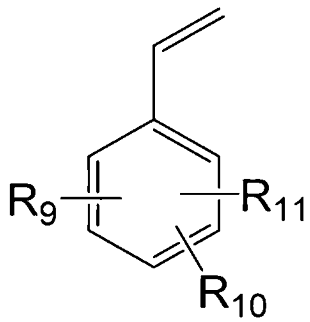

wherein in Chemical Formulas 1-1 to 1-3,
$R_6$ to $R_{11}$ are each independently an alkyl group having one or more carbon atoms.

11. The preparing method for microcarrier for cell culture of claim 9, wherein:
the compound represented by Chemical Formula 1 have a density of 0.92 g/cm$^3$ or less.

12. The preparing method for microcarrier for cell culture of claim 9, wherein:
the compound represented by Chemical Formula 1 is included in an amount of 51 parts by weight or more based on 100 parts by weight of the styrene-based monomer mixture.

13. The preparing method for microcarrier for cell culture of claim 9, wherein:
comprises a step of coating a cell attachment-inducing layer on the polystyrene-based particles.

14. A cell culture composition comprising cells and the microcarrier for cell culture of claim 1.

15. The cell culture composition of Claim 14, wherein:
the cell comprises at least one compound selected from the group consisting of the fibroblasts, epithelial cells, osteoblasts, chondrocytes, hepatocytes, human umbilical cord blood-derived cells, human bone marrow-derived

mesenchymal stem cells, CHO cells, kidney cells.

**16.** The cell culture composition of Claim 14, wherein:
the difference in apparent density between the microcarrier for cell culture and the cells is 0.02 g/cm$^3$ or more and 0.20 g/cm$^3$ or less.

# INTERNATIONAL SEARCH REPORT

International application No. **PCT/KR2025/095355**

## A. CLASSIFICATION OF SUBJECT MATTER

**C12N 5/00**(2006.01)i

According to International Patent Classification (IPC) or to both national classification and IPC

## B. FIELDS SEARCHED

Minimum documentation searched (classification system followed by classification symbols)

C12N 5/00(2006.01); C08F 12/08(2006.01); C12M 1/12(2006.01); C12N 11/00(2006.01); C12N 11/08(2006.01); C12N 5/06(2006.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal), STN (Registry, Caplus, Marpat), Google & keywords: 단량체 (monomer), 스티렌 (styrene), 세포 배양 (cell culture), 마이크로 캐리어 (micro carrier)

## C. DOCUMENTS CONSIDERED TO BE RELEVANT

| Category* | Citation of document, with indication, where appropriate, of the relevant passages | Relevant to claim No. |
|---|---|---|
| X | US 6214618 B1 (HILLEGAS, W. J. et al.) 10 April 2001 (2001-04-10)<br>abstract; claims 1-13; columns 4, 5; figure 1A | 1-16 |
| X | US 4994388 A (HILLEGAS, W. J. et al.) 19 February 1991 (1991-02-19)<br>claims 1-14; column 6 | 1-16 |
| A | CN 1289842 A (INST. OF CHEMICAL METALLURGY, CHINESE ACADEMY OF SCIENCES) 04 April 2001 (2001-04-04)<br>claims 1-10 | 1-16 |
| A | KR 10-2021-0011340 A (LG CHEM, LTD.) 01 February 2021 (2021-02-01)<br>claims 1-18 | 1-16 |
| A | KR 10-2022-0036336 A (LG CHEM, LTD.) 22 March 2022 (2022-03-22)<br>claims 1-20 | 1-16 |

☐ Further documents are listed in the continuation of Box C.  ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **19 August 2025** | **20 August 2025** |

| Name and mailing address of the ISA/KR | Authorized officer |
|---|---|
| **Korean Intellectual Property Office**<br>**Government Complex-Daejeon Building 4, 189 Cheongsa-ro, Seo-gu, Daejeon 35208** | |
| Facsimile No. **+82-42-481-8578** | Telephone No. |

Form PCT/ISA/210 (second sheet) (July 2022)

**INTERNATIONAL SEARCH REPORT**
**Information on patent family members**

International application No.
**PCT/KR2025/095355**

| Patent document cited in search report | Publication date (day/month/year) | Patent family member(s) | Publication date (day/month/year) |
|---|---|---|---|
| US 6214618 B1 | 10 April 2001 | None | |
| US 4994388 A | 19 February 1991 | None | |
| CN 1289842 A | 04 April 2001 | None | |
| KR 10-2021-0011340 A | 01 February 2021 | EP 3889252 A1 | 06 October 2021 |
| | | JP 2022-521518 A | 08 April 2022 |
| | | JP 7246814 B2 | 28 March 2023 |
| | | US 2022-0081684 A1 | 17 March 2022 |
| | | WO 2021-015547 A1 | 28 January 2021 |
| KR 10-2022-0036336 A | 22 March 2022 | EP 4134425 A1 | 15 February 2023 |
| | | JP 2023-531012 A | 20 July 2023 |
| | | JP 7542909 B2 | 02 September 2024 |
| | | US 2023-0250391 A1 | 10 August 2023 |
| | | WO 2022-059980 A1 | 24 March 2022 |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- KR 1020240067363
- KR 1020250066732